# EUROPÄISCHE PATENTSCHRIFT

(11) **EP 0 635 246 B1**
(45) Veröffentlichungstag und Bekanntmachung des Hinweises auf die Patenterteilung: **04.11.1998**
(21) Anmeldenummer: 94110678.3
(22) Anmeldetag: 08.07.1994
(51) Int. Cl.: A61F 2/44, A61B 17/58, F16B 13/06, A61F 2/30

(54) **Wirbelkörperfusionsdübel**
Dowel for fusing vertebral bodies
Cheville pour la fusion de corps vertébraux

(30) Priorität: 19.07.1993 DE 4323956
(43) Veröffentlichungstag der Anmeldung: 25.01.1995
(73) Patentinhaber: ESKA Implants GmbH & Co., 23556 Lübeck (DE)
(72) Erfinder: Grundei, Hans, Dr., D-23558 Lübeck (DE)
(74) Vertreter: Fuchs Mehler Weiss & Fritzsche

(56) Entgegenhaltungen:
- EP-A- 0 298 235
- EP-A- 0 385 930
- WO-A-90/00037
- WO-A-92/14423
- WO-A-93/11717
- AT-B- 393 214
- FR-A- 2 653 660
- US-A- 5 059 193

## Beschreibung

Die Erfindung betrifft einen Wirbelkörperfusionsdübel zur knöchernen Fusion zweier benachbarter Wirbelkörper der menschlichen Wirbelsäule.

Bekannt sind Wirbelkörperimplantate allgemein zur Fusion zweier Wirbelkörper. Klassisch ist beispielsweise das sogenannte Keystone-Implantat. Aus jüngerer Zeit bekannt sind derartige Implantate beispielsweise aus der DE 40 12 622 C1 und der DE 91 01 603 U1. Die darin beschriebenen Implantate sind zwar sehr vorteilhaft, jedoch in erster Linie bestimmt für Einsatzfälle, bei denen ganze Wirbelkörper zum Teil reseziert und ersetzt werden müssen. Sie sind daher von ihrer Dimension allein schon recht ausladend und mechanisch kompliziert, was allerdings in den für sie vorgesehenen Applikationsfällen auch gerechtfertigt ist. Anders hingegen sieht es bei kleineren Defekten der Wirbelkörper bzw. der dazwischen liegenden Bandscheibe aus. Bei im Gegensatz zu Indikationsfällen, in denen Implantate der oben beschriebenen Art zum Einsatz kommen, medizinisch recht einfach gelagerten Fällen ist es per se nicht angezeigt, mit derartig großen Implantaten operativ Abhilfe zu schaffen. Hier spielt selbstverständlich die medizinische Philosophie eine Rolle, so viel wie möglich körpereigene Teile im Körper zu belassen und so wenig lädierte Körperteile wie nötig zu entfernen.

Ein Wirbelsäulenimplantat ist bekannt geworden aus der EP-0 260 044 A1. Gemaß einer Ausführungsform ist dort vorgesehen, einen im wesentlichen zylindrischen Körper zweigeteilt aus zwei Halbschalen zu bilden. In deren Inneren ist eine Gewindespindel mit gegenläufigen Gewinden gelagert, auf der zwei Nocken aufgeschraubt sind. Die beiden Nocken sind im nicht-verspannten Zustand des Implantates untergebracht in entsprechend ausgebildeten Taschen in den beiden Zylinderhälften. Auf eine Drehung der Gewindespindel zum Verspannen des Implantates in der Wirbelsäule werden diese Nocken aus den besagten Taschen herausgedrängt, so daß die beiden Hälften des zylindrischen Körpers auseinandergespreizt werden. Es findet demnach eine Parallelverschiebung der Wirbelkörper statt, an denen die beiden Zylinderhälften anliegen.

Als problematisch jedoch erweist es sich, daß im Falle der Anbringung einer Bohrung bzw. eines Kanals in einem Wirbelkörper das noch nicht extrahierte Implantat zwar dort hinein gesetzt werden kann, wenn jedoch die Gewindespindel gedreht wird, so daß sich die beiden Zylinderhälften parallel voneinander entfernen, so findet das Implantat lediglich durch einen Preßfit Halt in der Wirbelsäule. Am Eingang des Kanals bzw. der eingebrachten Bohrung findet sich bei dem Inplantat derselbe Querschnitt wie am Ende des- bzw. derselben, nämlich ein ovaler Querschnitt. Aufgrund von Belastungen der Wirbelsäule im täglichen Leben kann es nicht ausgeschlossen werden, daß der Preßfit des Implantates gelockert wird und dem Patienten die Gefahr eines Herausrutschens des Implantates aus der Bohrung bzw. aus dem Kanal erwächst. Dies ist nicht akzeptabel.

Gemäß einem späteren Vorschlag gemäß der WO92/14423, in der auch das vorerwähnte Implantat Erwähnung findet, wird dem Herausrutschen aus dem Kanal in den Wirbelkörpern versucht entgegenzuwirken, indem das Implantat nach Einsetzen in den Kanal in seiner Mitte in seinem Querschnitt vergrößert wird. Am Anfang und am Ende des Kanals bzw. der Bohrung finden sich die kleinsten Querschnitte während der Querschnitt des Implantates nach der Implantation und dem Spannen in der Mitte am größten ist. Es trifft zu, daß dem Herausrutschen hierdurch wirksam entgegengewirkt wird. Als nachteilig wird allerdings angesehen, daß die größte Erweiterung bzw. größte Spreizung des Implantates nicht am sozusagen eigentlichen Ort des Geschehens vorgenommen wird, sondern weit dahinter gelegen. Der Ort nämlich, wo ein Implantat seine therapeutische Wirkung entfalten sollte, ist der Übergangsbereich vom knöchernen Material eines Wirbelkörpers zur Bandscheibe, welche bei der indizierten Operation und Einsatz eines solchen Implantates beschädigt ist. Im Grenzbereich zwischen knöchernem Material und der beschädigten Bandscheibe ist dafür Sorge zu tragen, daß die Spreizung in diesem Bereich am größten ist. Dies vermag keines der bekannten Implantate zu leisten.

Vor diesem Hintergrund ist es die Aufgabe der vorliegenden Erfindung, ein Implantat zur knöchernen Fixation zweier benachbarter Wirbelkörper der menschlichen Wirbelsäule anzugeben, welches diese Wirbelkörper dauerhaft miteinander verbindet und gleichzeitig für die maximale Spreizung der benachbarten Wirbelkörper im Bereich der Grenzfläche zwischen knöchernen Material und dem Material der Bandscheibe sorgt.

Ausgehend von dem Implantat gemäß der erwähnten WO92/14423 wird diese Aufgabe durch das Implantat mit den kennzeichnenden Merkmalen des Anspruchs 1 gelöst. Weitere vorteilhafte Ausgestaltungen ergeben sich aus den Unteransprüchen. Demnach wird vorgeschlagen, das Implantat als eine Art Dübel auszubilden. Wie das Implantat gemäß der vorerwähnten Druckschrift besteht dieses aus einer Hülse und einer die Hülse durchsetzenden Schraube, die an der bis auf eine Durchbohrung für die Schraube geschlossenen proximalen Stirnseite der Hülse in diese eingesetzt ist. Erfindungsgemäß ist nun vorgesehen, daß wenigstens zwei in der Hülse von ihrem distalen Ende zum proximalen Ende über im wesentlichen 2/3 ihrer Länge verlaufenden Schlitze sowie eine mit der Schraube verschraubbare Mutter vorgesehen sind, die von der distalen Seite in die Hülse greift, wobei die Mutter einen kreisförmigen Querschnitt und ausgehend von einem zylindrischen Teil eine sich zum offenen Hülsenende konisch erweiternde Mantelfläche aufweist und der Durchmesser des zylindrischen Abschnittes in etwa dem Innendurchmesser der Hülse entspricht. Das bewirkt, daß der zylindrische Abschnitt in der Hülse zunächst geführt werden kann.

Wie einsichtig ist, wird bei weiterem Anziehen der Schraube die speziell ausgebildete Mutter noch weiter in die Hülse hinein gezogen. Ist die Mutter so weit in die Hülse hineingezogen, daß nun nachfolgend die sich konisch aufweitende Mantelfläche an der distalen Stirnseite der Hülse ansteht, und wird die Schraube noch weiter gedreht, so wird die Hülse in diese hineingezogen unter Aufweitung der Wandungssegmente der Hülse. Dadurch kommt der bekannte Sperreffekt eines Dübels zustande.

Im Gegensatz zu der gattungsbildenden Druckschrift WO92/14423 nimmt der erfindungsgemäße Wirbelkörperfusionsdübel nach dem Spannen eine trapez- oder V-förmige Form an, wobei die maximale Spreizung an seinem distalen Ende erzielt wird, nämlich genau dort, wo die Bohrung bzw. der Kanal in den Wirbelkörpern im Grenzbereich zu dem Bandscheibenmaterial endet.

Klar ist, daß die Mutter aus einem entsprechend härterem Material bestehen muß, um daß körperverträgliche Metall der Hülse aufweiten zu können.

Die durch die Aufweitung der Wandungssegmente erzielte Primärfixation gleicht einem Preßsitz. Für eine dauerhafte Sekundärfixation und damit eine dauerhafte Fusion beider Wirbelkörper ist vorteilhaft vorgesehen, daß die äußere Mantelfläche der Hülse mit einer dreidimensionalen offenmaschigen Raumnetzstruktur belegt ist, in welche Knochentrapekel einwachsen können und so für einen dauerhaften Halt sorgen. Die Herstellung einer derartigen Raumnetzstruktur ergibt sich beispielsweise aus der DE 41 06 971 C1.

Damit ein unbeabsichtigtes Lösen der Mutter von der Schraube vermieden wird, ist vorzugsweise vorgesehen, daß die Mutter vor einem Abdrehen von der Schraube mittels einer Verdrehsicherung gesichert ist. Diese Verdrehsicherung kann beispielsweise aus einer Radialnut in der Schraube und einem darin eingreifenden Sprengring bestehen.

Die Handhabung des erfindungsgemäßen Implantats wird in situ noch dadurch deutlich vereinfacht, daß gemäß einer vorteilhaften Ausführungsform einer der Längsschlitze in der Hülse im distalen Bereich als Führungsnut ausgebildet ist, und zwar für einen an der Mutter angebrachten, als Verdrehsicherung wirkender Bolzen. Hierdurch wird die Mutter vor einem Mitdrehen anstelle einer Aufweitung der Wandungssegmente gehindert.

Die Erfindung wird anhand eines Ausführungsbeispiels gemäß der Zeichnungsfiguren näher erläutert. Hierbei zeigt:
- Fig. 1: eine Seitenansicht auf das erfindungsgemäße Implantat,
- Fig. 2: eine Schnittansicht des Implantats aus Fig. 1, und
- Fig. 3: eine Aufsicht auf das Implantat aus den Figuren 1 und 2 von rechts gesehen.

Nachfolgend sind gleiche Teile mit den gleichen Bezugszeichen versehen.

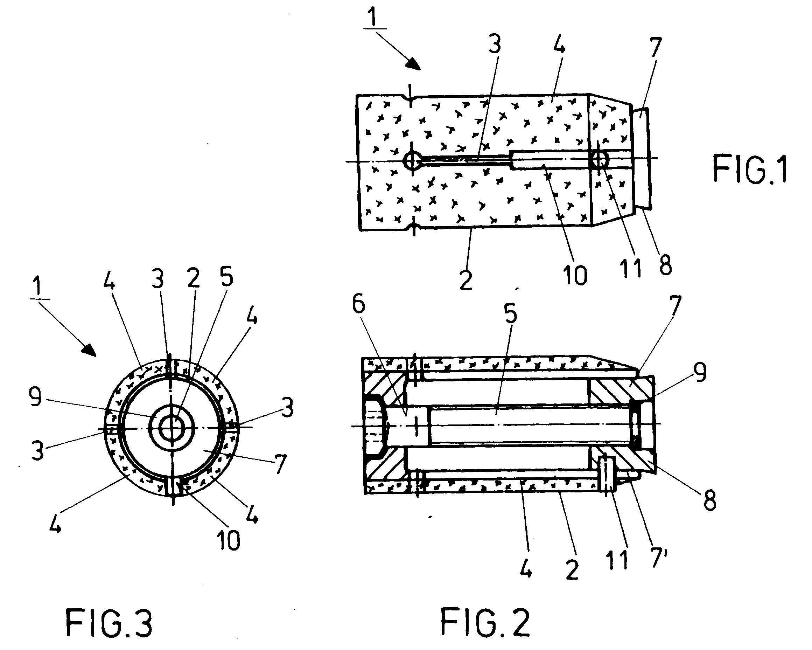

Einen ersten Überblick verschafft Fig. 1. Der darin dargestellte Wirbelkörperfusionsdübel 1 besteht aus einer Hülse 2, die hier als mit einer dreidimensionaler offenmaschigen Raumnetzstruktur 4 versehen dargestellt ist. Die Hülse 2 weist vorliegend vier Längsschlitze 3 (Fig. 3) auf, welche im dargestellten Ausführungsbeispiel zu vierfünfteln über ihre Länge verlaufen. Einer der drei Schlitze 3 ist im distalen Bereich erweitert zu einer Führungsnut 10, in der ein Bolzen 11 geführt ist, der mit der Mutter 7 verbunden ist, welche in das distale Ende der Hülse geschoben und mit der die die Hülse 2 durchsetzende Schraube 5 verschraubt ist. Die proximale Seite der Hülse 2 (in Fig. 2 links) ist bis auf die Durchbohrung 6 für die Schraube 5 geschlossen. Die Mutter 7 weist einen zylindrischen Teil 7' auf, der in seinem Durchmesser in etwa dem Innendurchmesser der Hülse 2 entspricht. Diesem zylindrischen Teil 7' der Mutter 7 schließt sich zum distalen Ende hin ein Abschnitt mit sich konisch erweiternder Mantelfläche 8 an.

Die Wirkungsweise des Dübels ist folgende:

Nach Anbringung einer geeigneten Ausfräsung in den beiden zu fusionierenden Wirbelkörper wird der vormontierte, noch in etwa zylindrische Dübel mit Mutter 7 und Schraube 5 in die Ausfräsung gesetzt und sodann die Schraube 5 mittels eines Schraubendrehers betätigt, woraufhin die Hülse 7 weiter in das Hülseninnere gezogen wird. Erreicht der Bereich mit sich konisch erweiternder Mantelfläche 8 die Stirnseite der Hülse, so bewirkt eine weitere Drehung der Schraube ein Sichaufspreizen der Wandungselemente der Hülse. Der Dübel spreizt sich und sorgt für die fusionierende Wirkung.

Nach der Implantation wachsen Knochentrapekel in die dreidimensionale offenmaschigen Raumnetzstruktur 4 ein und sorgen so für eine stabile Langzeitfixation bzw. -fusion.

## Patentansprüche

1. Wirbelkörperfusionsdübel (1), bestehend aus einer Hülse (2) und einer die Hülse durchsetzenden Schraube, die an der bis auf eine Durchbohrung (6) für die Schraube geschlossenen proximalen Stirnseite der Hülse in diese eingesetzt ist,
gekennzeichnet durch,
wenigstens zwei in der Hülse (2) von ihrem distalen Ende zum proximalen Ende über im wesentlichen 2/3 ihrer Länge verlaufenden Schlitze (3), und eine mit der Schraube (5) verschraubbare Mutter (7), die von der distalen Seite in die Hülse (2) greift, wobei die Mutter (7) einen kreisförmigen Querschnitt und ausgehend von einem zylindrischen Teil (7') eine sich zum offenen Hülsenende konisch erweiternde Mantelfläche (8) aufweist und der Durchmesser des zylindrischen Abschnitts (7') in etwa dem Innendurchmesser der Hülse (2) entspricht.

2. Wirbelkörperfusionsdübel nach Anspruch 1, bei dem die äußere Mantelfläche der Hülse (2) mit einer dreidimensionalen offenmaschigen Raumnetzstruktur (4) belegt ist.

3. Wirbelkörperfusionsdübel nach Anspruch 1 oder 2, bei dem die Mutter (7) vor einem Abdrehen von der Schraube (5) mittels einer Verdrehsicherung (9) gesichert ist.

4. Wirbelkörperfusionsdübel nach einem der Ansprüche 1 bis 3, bei dem einer der Schlitze (3) im distalen Bereich als Führungsnut (10) ausgebildet ist für einen an der Mutter (7) angebrachten, als Verdrehsicherung wirkender Bolzen (11).

## Claims

1. Vertebral body fusion dowel (1), consisting of a sleeve (2) and a screw penetrating the sleeve, which is inserted into the sleeve at the proximal end face of the latter which is closed except for a bore (6) for the screw, characterised by at least two slots (3) extending in the sleeve (2) from its distal end to the proximal end over essentially 2/3 of its length, and a nut (7) which can be screwed using the screw (5) and engages in the sleeve (2) from the distal side, wherein the nut (7) has a circular cross-section and, starting from a cylindrical part (7'), a shell surface (8) expanding conically towards the open sleeve end, and the diameter of the cylindrical section (7') corresponds approximately to the internal diameter of the sleeve (2).

2. Vertebral body fusion dowel according to claim 1, in which the outer shell surface of the sleeve (2) is covered with a three-dimensional open-mesh spatial net structure (4).

3. Vertebral body fusion dowel according to claim 1 or 2, in which the nut (7) is secured by means of a retention device (9) before wringing off from the screw (5).

4. Vertebral body fusion dowel according to one of claims 1 to 3, in which one of the slots (3) is designed as a guide groove (10) in the distal region for a bolt (11) attached to the nut (7) and acting as a retention device.

## Revendications

1. Cheville de fusion de corps de vertèbres (1) comprenant une douille (2) et une vis traversant la douille, laquelle vis est engagée sur la face frontale proximale de la douille qui est fermée, à l'exception d'un perçage (6) pour la vis, caractérisée par au moins deux fentes (3) s'étendant de l'extrémité distale vers l'extrémité proximale de la douille (2) sur sensiblement 2/3 de sa longueur, et par un écrou (7) pouvant être vissé sur la vis (5) et s'engageant dans la douille (2) depuis le côté distal, l'écrou (7) présentant une section circulaire et, depuis une partie cylindrique (7'), une surface latérale (8) évasée coniquement en direction de l'extrémité ouverte de la douille, le diamètre de la section cylindrique (7') correspondant approximativement au diamètre intérieur de la douille (2).

2. Cheville de fusion de corps de vertèbres selon la revendication 1, dans laquelle la surface latérale extérieure de la douille (2) est revêtue d'une structure réticulée tridimensionnelle à mailles ouvertes (4).

3. Cheville de fusion de corps de vertèbres selon la revendication 1 ou 2, dans laquelle l'écrou (7) est protégé d'un dévissage de la vis (5) à l'aide d'un dispositif de blocage en rotation (9).

4. Cheville de fusion de corps de vertèbres selon l'une des revendications 1 à 3, dans laquelle l'une des fentes (3) est configurée comme encoche de guidage (10) dans la zone distale pour un boulon (11) disposé sur l'écrou (7) et servant de dispositif de blocage en rotation.
